(19) Europäisches Patentamt
European Patent Office
Office européen des brevets

(11) Publication number: 0 222 912 A1

(12) # EUROPEAN PATENT APPLICATION

published in accordance with Art. 158(3) EPC

(21) Application number: 85902644.5

(22) Date of filing: 24.05.85

Data of the international application taken as a basis:

(86) International application number: PCT/JP 85/00286

(87) International publication number: WO 85/05623 (19.12.85 85/27)

(51) Int. Cl.⁴: C 07 J 51/00, A 61 K 31/575

(30) Priority: 25.05.84 JP 106123/84

(43) Date of publication of application: 27.05.87 Bulletin 87/22

(84) Designated Contracting States: BE CH DE FR GB IT LI SE

(71) Applicant: ZERIA SHINYAKU KOGYO KABUSHIKI KAISHA, 10-11, Nihonbashi Kofune-cho, Chuo-ku Tokyo 103 (JP)

(72) Inventor: INAGAKI, Tetsuya, 2-9-18, Daitakubo Urawa-shi, Saitama-ken 336 (JP)
Inventor: AOKI, Hidemi, (305) Hairaku-Warabi 6-3-11, Tsukagoshi, Warabi-shi Saitama-ken 335 (JP)
Inventor: AIKAWA, Hiroyasu, 661-8, Miyamae-cho Omiya-shi, Saitama-ken 330 (JP)
Inventor: SAKAMOTO, Koji, 18, Daikyo-cho Shinjuku-ku, Tokyo 160 (JP)

(74) Representative: LOUIS, PÖHLAU, LOHRENTZ & SEGETH, Kesslerplatz 1 P.B. 3055, D-8500 Nürnberg (DE)

(54) **AGENT FOR TREATING ARTERIOSCLEROSIS.**

(57) An agent for treating arteriosclerosis, which contains as effective ingredient cycloartenol ferulate, a component of γ-oryzanol. This agent shows excellent effects of lowering a total cholesterol level, a free cholesterol level, a neural fat level, and a phospholipid level, and raising an HDL-cholesterol level (believed to be an arteriosclerosis alleviating factor) to remarkably reduce arteriosclerosis index with less liver troubles.

EP 0 222 912 A1

# SPECIFICATION

## TECHNICAL FIELD

This invention relates to an antiarteriosclerotic agent which comprises cycloartenol ferulate as an active component.

## BACKGROUND ART

Various pharmaceuticals have been used in treating arteriosclerosis. However these pharmaceuticals are frequently accompanied by side effects such as hepatic disorder, which is a serious problem in repeated administration thereof for a long period of time.

On the other hand, a pharmacological test with the uses of animals has revealed that γ-oryzanol is effective in decreasing lipids to a certain extent (Geriat. Mcd. 18: 519-524, 1980). However γ-oyzanol is a mixture of various triterpene alcohols. As a result of advances in analytical instruments, it has been reported that the alcohol portion of oryzanol comprises seven to ten triterpene alcohols (Kiso To Rinsho vol. 6, No. 7, 1479-1485, 1982; Yukagaku, 19, 298, 1970; and Kiso To Rinsho, 8(1), 35, 1974). In addition, it is believed that isolation and purification of each component is very difficult.

Thus there has been no report which succeeded in clarifying which of the components of γ-oryzanol could bring about the effect of decreasing lipids.

DISCLOSURE OF THE INVENTION

Objects of the present invention are to examine the active component of γ-oryzanol effective in decreasing lipids, to find the active substance which exerts such an effect and to clinically apply the same as an antiarteriosclerotic agent.

As a result of our examination on cycloartenol ferulate and its effect of decreasing lipids, we have found that cycloartenol ferulate exhibits a much higher effect of decreasing lipids than γ-oryzanol, thus completing the present invention.

The present invention relates to an antiarteriosclerotic agent which comprises cycloartenol ferulate as an active component.

Cycloartenol ferulate as mentioned herein may be isolated from γ-oryzanol and purified by the following method.

500 g of γ-oryzanol was dissolved in 1.5 to 2.0 ℓ of heated chloroform and methanol was added thereto until crystals separated out. These were heated again to be dissolved and then were allowed to stand at room temperature. Thus crystals separated out. The crystals thus obtained were washed with n-hexane. 100 g of the resulting crude cycloartenol ferulate was dissolved in chloroform, adsorbed by a reverse phase column packing and developed with methanol/chloroform/water at a volume ratio of 6 : 2 : 1. Main fractions were combined and the solvent was distilled off therefrom. The residue was recrystallized from chloroform/methanol at a volume ratio of 1 : 1 to thereby give 60 g of cycloartenol

ferulate.

Cycloartenol ferulate thus obtained was identified by high-performance liquid chromatography (HPLC), mass spectrometry (MS), infrared spectrum (IR) and nuclear magnetic resonance spectrum (NMR).

Thus, its HPLC shows a single peak as shown in Fig. 1.

Its MS shows a molecular ion peak (602) of cycloartenol ferulate as shown in Fig. 2.

Its IR shows a streching vibration of C=O at 1670 $cm^{-1}$, vibrations of methyl groups at 1470 to 1430 $cm^{-1}$ and at 1380 to 1350 $cm^{-1}$ and a characteristic pattern of vibration of a cyclopropane ring at 1020 $cm^{-1}$ as shown in Fig. 3.

Its NMR shows signals of a cyclopropane ring, an -OCH group, a hydroxyl group of phenol and ${}_{H}{>}C = C{<}^{H}$ at $\delta$values of 0.58 ppm, 3.92 ppm, 5.29 ppm, and 6.15 and 7.47 ppm, respectively. Furthermore it shows a signal of 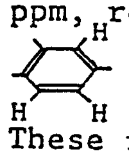 at 6.93 ppm.

These results prove that the product is cycloartenol ferulate.

Now the effect of decreasing lipids of cycloartenol ferulate will be described.

In order to examine the effect of decreasing lipids, male SD rats aged four weeks were classified into the following four groups: i.e. (1) an ordinary diet group (control); (2) a cholesterol diet group; (3) a cholesterol diet group to which cycloartenol ferulate was administered; and (4) a cholesterol diet group to which $\gamma$-oryzanol was administered.

Cycloartenol ferulate or $\gamma$-oryzanol was orally administered for 12 days repeatedly with the use of a metal probe. Each compound was administered in doses of 100 mg/kg, 500 mg/kg and 1000 mg/kg per animal a day. After each administration, blood of the animal was collected to determine free cholesterol (FC), triglyceride (TG) and phospholipids (PL) contained therein.

The results as shown in Figs. 5 to 12 clearly indicate that cycloartenol ferulate is much more effective as an antiarteriosclerotic agent than $\gamma$-oryzanol.

Thus, the results of examination on FC, TG and PL have reveald that each value of the group to which cycloartenol ferulate was administered was lower than that of the cholesterol diet group to a statistically significant extent and that the effect of cycloartenol ferulate is remarkably higher than that of $\gamma$-oryzanol (Figs. 5 to 7).

Basal and clinical studies have revealed that HDL-cholesterol (HDLC) is a factor contributing to prevention of arteriosclerosis and that increase of HDLC proves usefullness as an antiarteriosclerotic agent.

As shown in Fig. 8, it has been clarified that cycloartenol ferulate can increase HDLC to a statistically significant extent as compared with the cholesterol diet group. On the other hand, $\gamma$-oryzanol hardly exhibits this effect.

It is known in the art that a valuable antiarteriosclerotic agent can reduce arteriosclerosis

index ($\frac{TC - HDLC}{HDLC}$); wherein TC represents total cholesterol). As shown in Fig. 9, cycloartenol fèrulate remarkably lowers this index. In contrast thereto, $\gamma$-oryzanole hardly exhibits this effect. This fact suggests that the former is more useful as an antiarteriosclerotic agent than the latter.

As described previously, conventional antiarteriosclerotic agent are frequently accompanied by hepatic disorder as a side effect, which is a serious problem in repeated administration of the same. Thus GOT and GPT were determined to thereby examine the effects of the above agents on liver. It is generally known that an increase in these values points to hepatic disorder. As shown in Figs. 10 and 11, administration of cycloartenol ferulate in a larger dose resulted in no changes in GOT nor GPT. On the other hand, administration of $\gamma$-oryzanol in the same dose resulted in a significant increase in GPT. GOT also showed a tendency to increase in this case.

As shown in Fig. 12, cycloartenol ferulate inhibited an effect of suppressing the body weight gain of rats by the cholesterol diet, i.e. it accelerated the growth of animals. On the other hand, the growth accelerating effect of $\gamma$-oryzanol was very small.

These facts clearly indicate that cycloartenol ferulate is a useful pharmaceutical compound which exerts an excellent effect of decreasing lipids without any trouble in safety when administered repeatedly for a long period of time.

In order to examine the mechanism of the lipid

decreasing effect of cycloartenol ferulate; the effect of the same on hyperlipemia induced by a surfactant (Triton WR-1339) was confirmed.

The test was conducted with male SD rats aged six weeks. 300 mg/kg of Triton WR-1339 was intravenously injected to each rat of the first group to thereby induce hyperlipemia (Triton control group), while 10 mg/kg of cycloartenol ferulate or 10 mg/kg of γ-oryzanol was intravenously injected simultaneously with 300 mg/kg of Triton WR-1339 to each rat of other groups, thus examining the effect of decreasing lipids. Blood of each animal was collected 43 hours after the intravenous injection.

As shown in Figs. 13 to 15, the group to which cycloartenol ferulate was administered showed significant decreases of TC (Fig. 13), FC (Fig. 14) and TG (Fig. 15) 43 hours after the intravenous injection of Triton WR-1339 as compared with the Triton control group and the γ-oryzanol administered group.

This result proves that cycloartenol ferulate exhibits a catabolic excretion effect on lipids in blood. It is believed that the lipids decreasing effect of γ-oryzanol is based on a mechanism of inhibiting absorption of lipids through intestine. However it has been proved that cycloartenol ferulate is effective not only in inhibiting absorption of lipids through intestine but also in accelerating catabolic excretion of lipids in blood as it transfers into blood.

Now the acute toxicity of cycloartenol ferulate will be described.

In order to examine the acute toxicity of cycloartenol ferulate, it was orally administered to five dd mice and five SD rats. As a result, no animal died nor showed any toxic symptom when 5 g/kg of cycloartenol ferulate was orally administered to these animals.

## BRIEF DESCRIPTION OF THE DRAWINGS

Figs. 1 to 4 are various spectra of cycloartenol ferulate, which is an active component of the antiarteriosclerotic agent of the present invention, where Fig. 1 is a pattern chart obtained by high-performance liquid chromatography, Fig. 2 is a mass spectrum, Fig. 3 is an infrared spectrum and Fig. 4 is a nuclear magnetic reasonance spectrum. Figs. 5 to 12 are given to show the pharmacological effects of the antiarteriosclerotic agent of the present invention on choloesterol diet, wherein Fig. 5 shows a comparison of the effects of decreasing free cholesterol, Fig. 6 shows a comparison of the effects of decreasing triglyceride, Fig. 7 shows a comparison of the effects of decreasing phospholipids, Fig. 8 shows a comparison of HDL-cholesterol levels, Fig. 9 shows a comparison of arteriosclerosis indexes, Fig. 10 shows a comparison of GPT values, Fig. 11 shows a comparison of GOT values and Fig. 12 shows a comparison of body weight gain. Figs. 13 to 15 are given to show the pharmacological effect of the antiarteriosclerotic agent of the present invention on hyperlipemia induced by Triton, where Fig. 13 shows a comparison of the effects of decreasing total cholesterol, Fig. 14 shows a comparison of the effects

of decreasing free cholesterol and Fig. 15 shows a comparison of decreasing triglyceride.

BEST MODE FOR CARRYING OUT THE INVENTION

When the antiarteriosclerotic agent of the present invention is used in treating arteriosclerosis, 0.01 to 5 g, preferably 0.1 to 5 g, of cycloartenol ferulate as the active component is orally administered to an adult a day.

It may be orally administered in a conventional form such as powder, fine granules, granules, capsule or syrup which may be prepared in a conventional method.

CAPABILITY OF EXPLOITATION IN INDUSTRY

As described above, the antiarteriosclerotic agent of the present invention is significantly useful since cycloartenol ferulate, which is an active component thereof, exerts excellent effects of decreasing total cholesterol, free cholesterol, triglyceride and phospholipids and of enhancing the HDL-cholesterol level, which is believed to be an improving factor of arteriosclerosis, to thereby significantly lower the arteriosclerosis index without accompanying any hepatic disorder.

WHAT IS CLAIMED IS:

1. An antiarteriosclerotic agent comprising cycloartenol ferulate as an active component.

Fig. 1

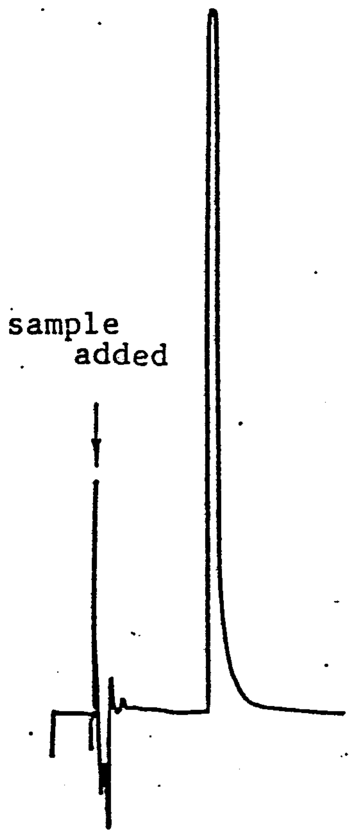

Fig. 2

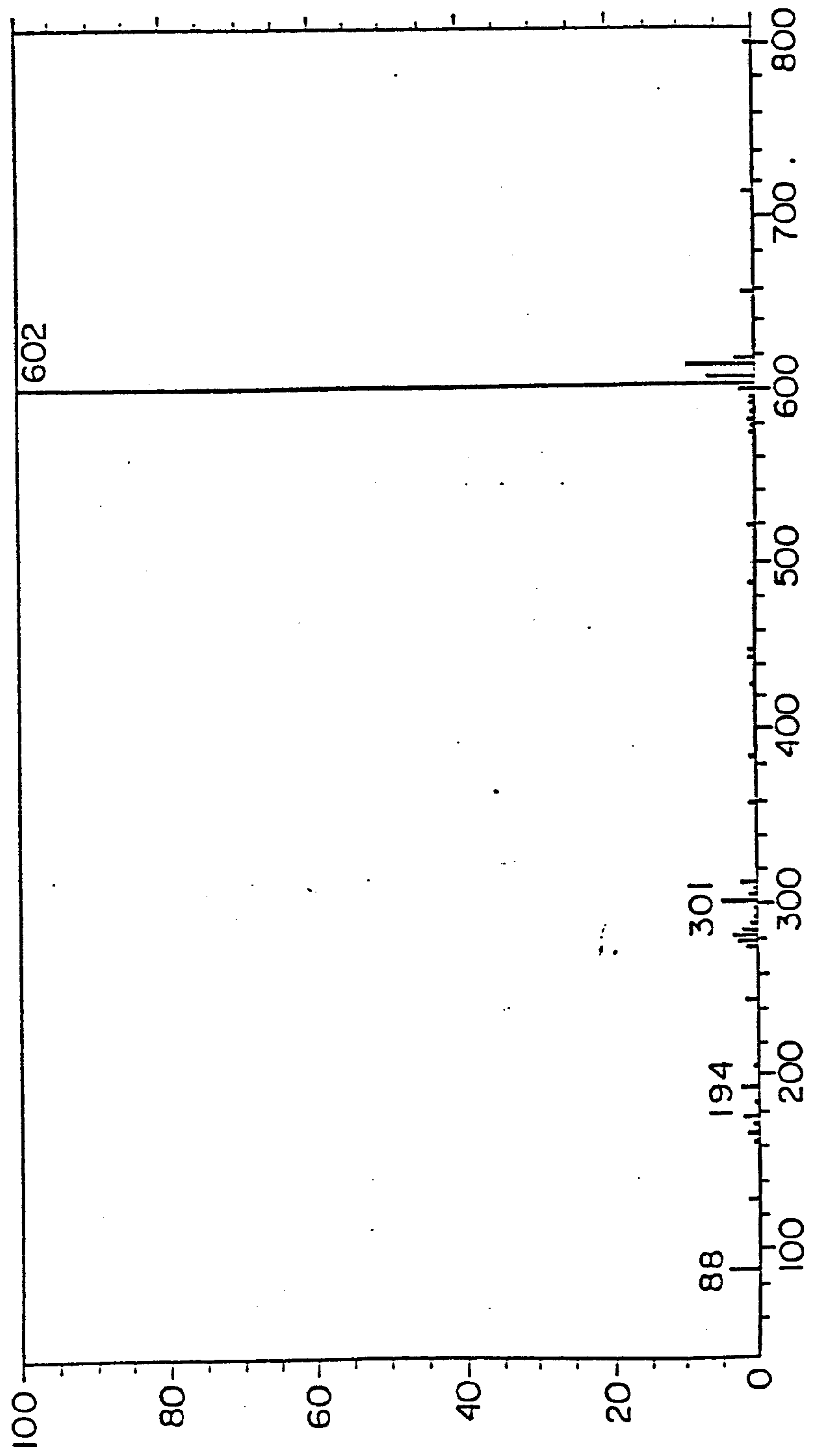

Fig. 3

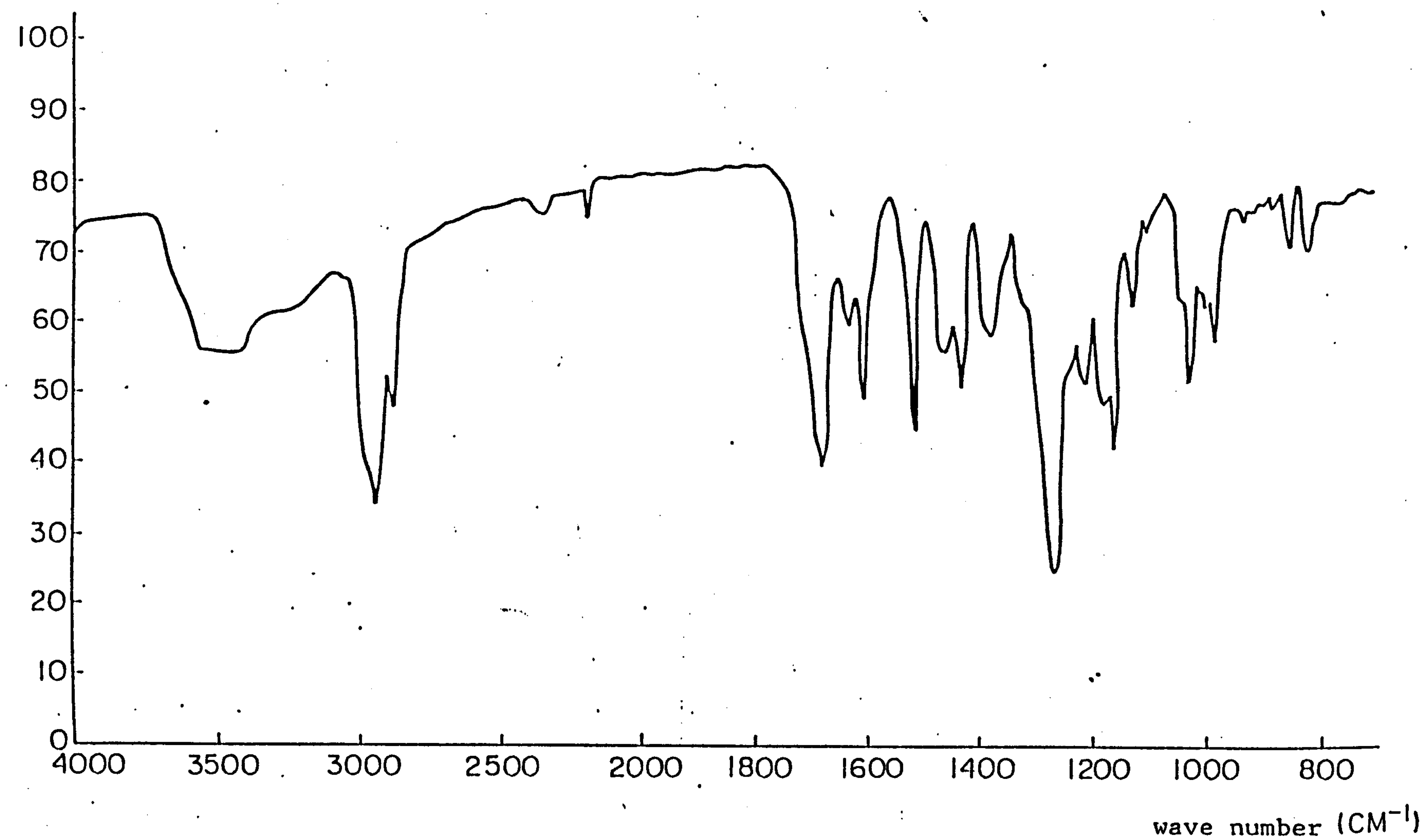
100
90
80
70
60
50
40
30
20
10
0
4000
3500
3000
2500
2000
1800
1600
1400
1200
1000
800
wave number (CM⁻¹)

4/10

Fig. 4

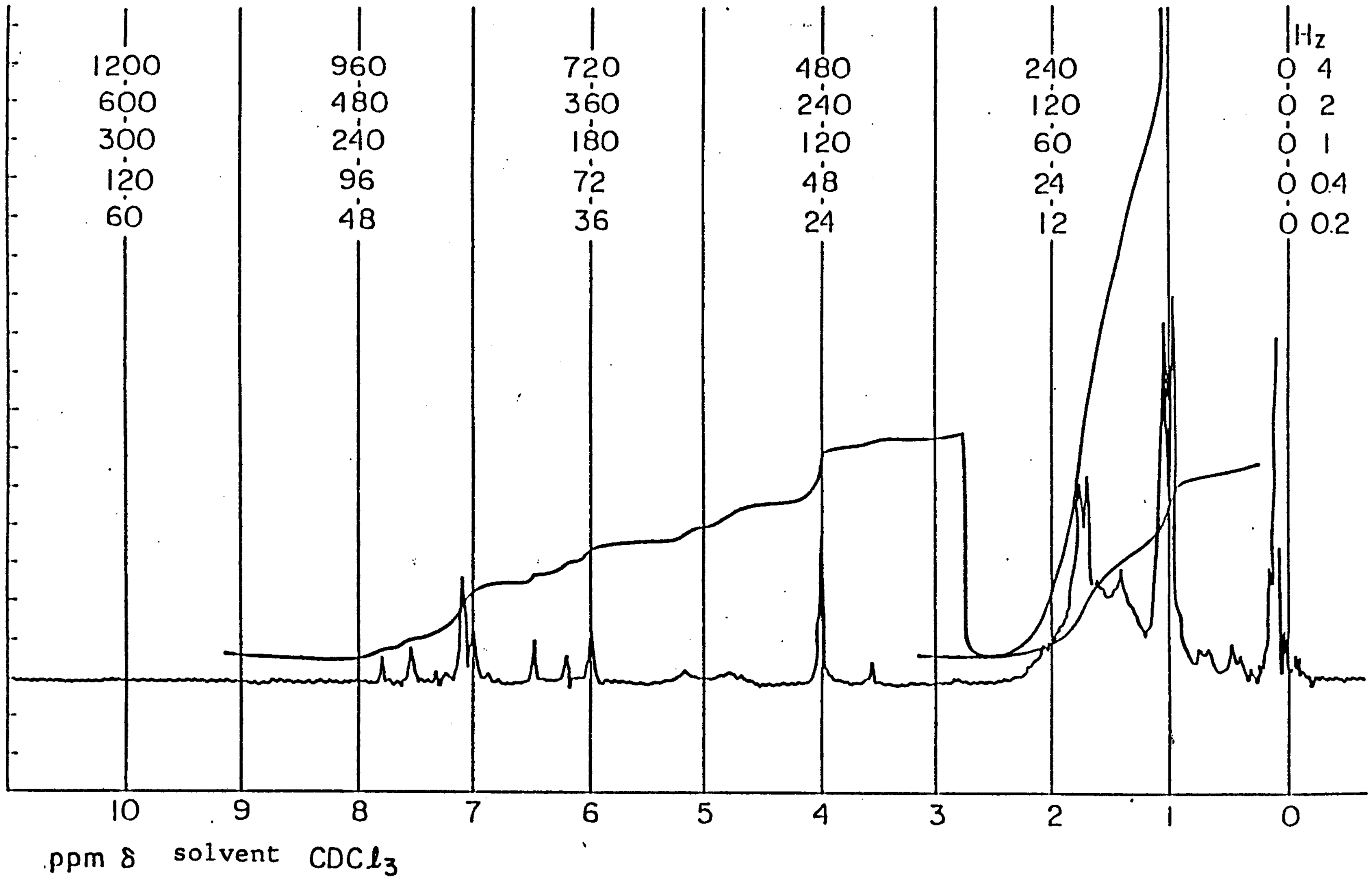
Hz
1200 600 300 120 60
960 480 240 96 48
720 360 180 72 36
480 240 120 48 24
240 120 60 24 12
0 4
0 2
0 1
0 0.4
0 0.2
10 9 8 7 6 5 4 3 2 1 0
ppm δ solvent $CDCl_3$

# Fig. 5

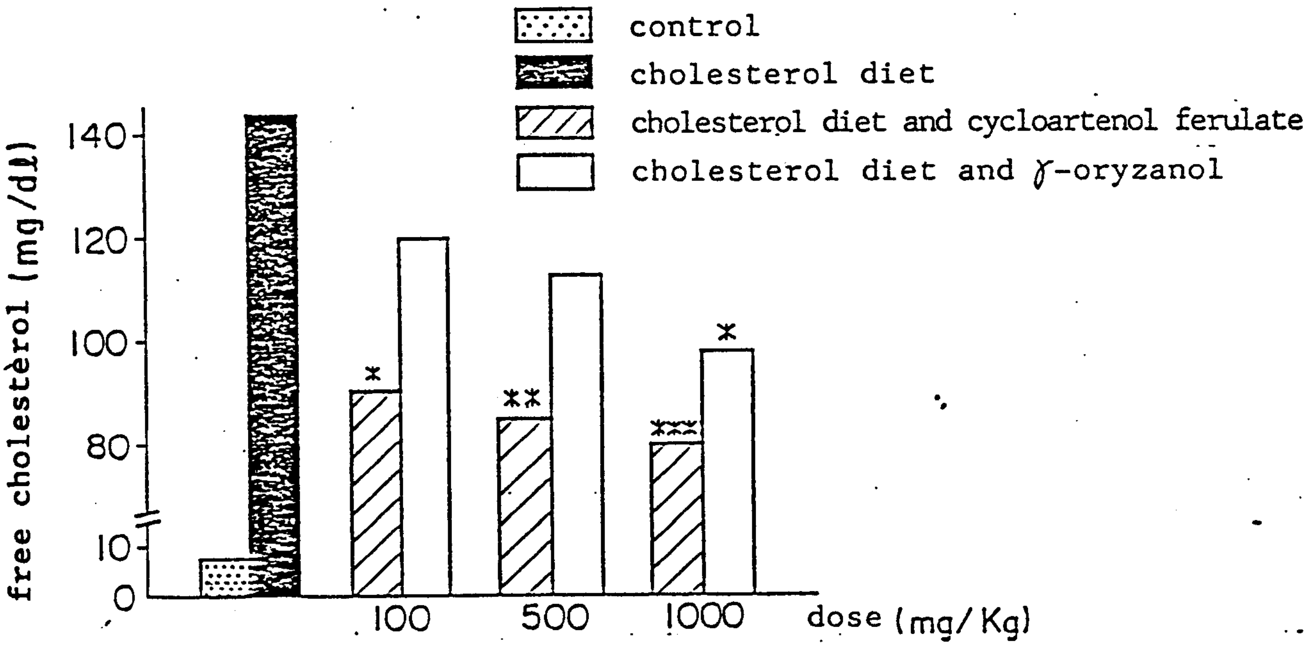


significant difference from cholesterol diet
* P<0.05 ** P<0.01 *** P<0.001

# Fig. 6

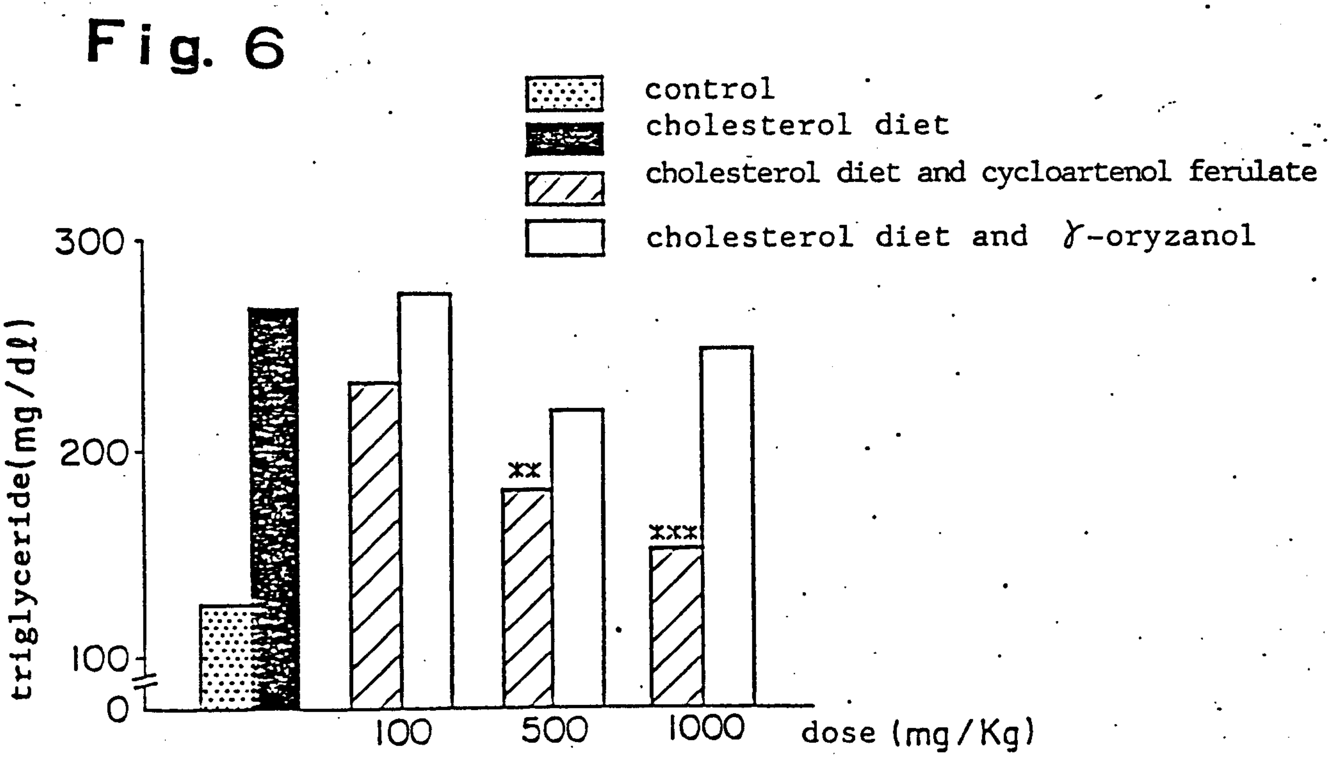


significant difference from cholesterol diet
** P<0.01 *** P<0.001

Fig. 7

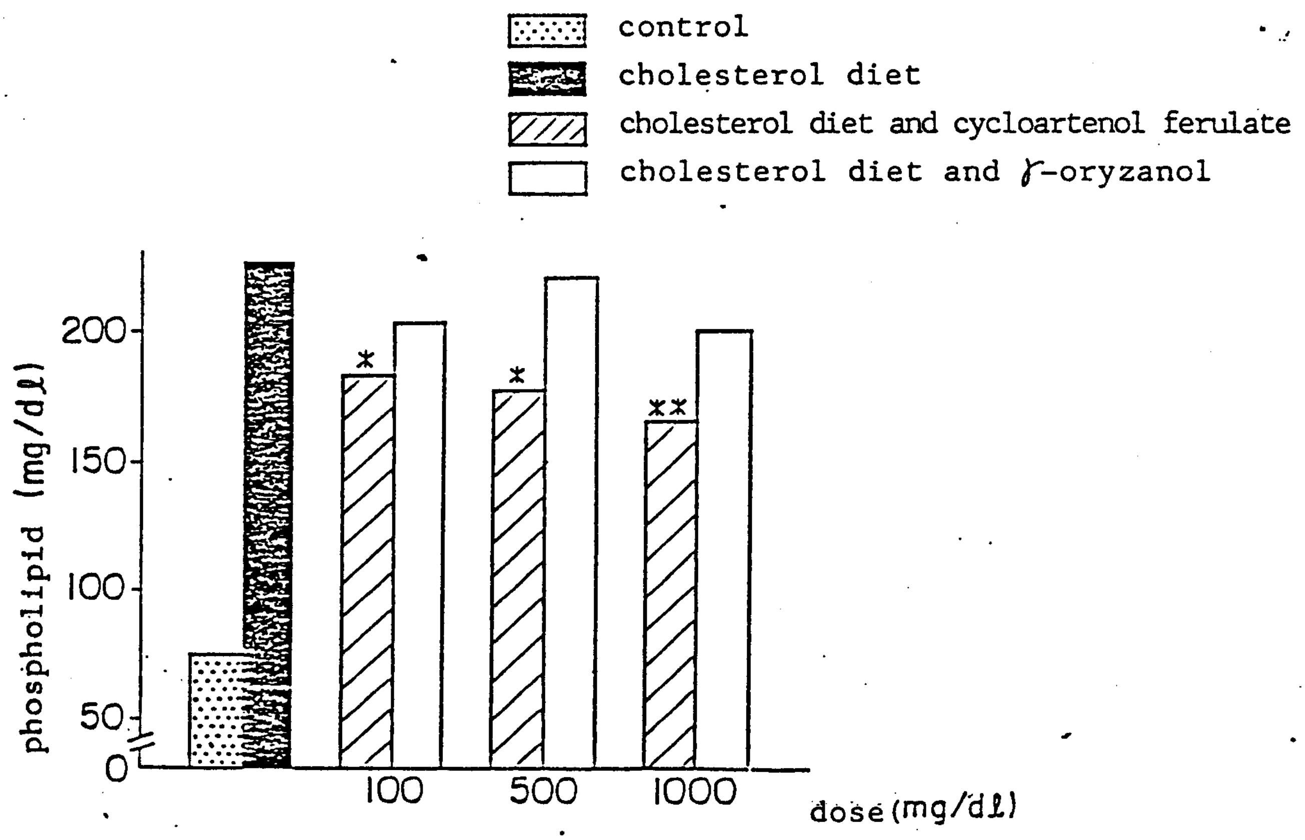


significant difference from cholesterol diet
*$P < 0.05$ **$P < 0.01$

Fig. 8

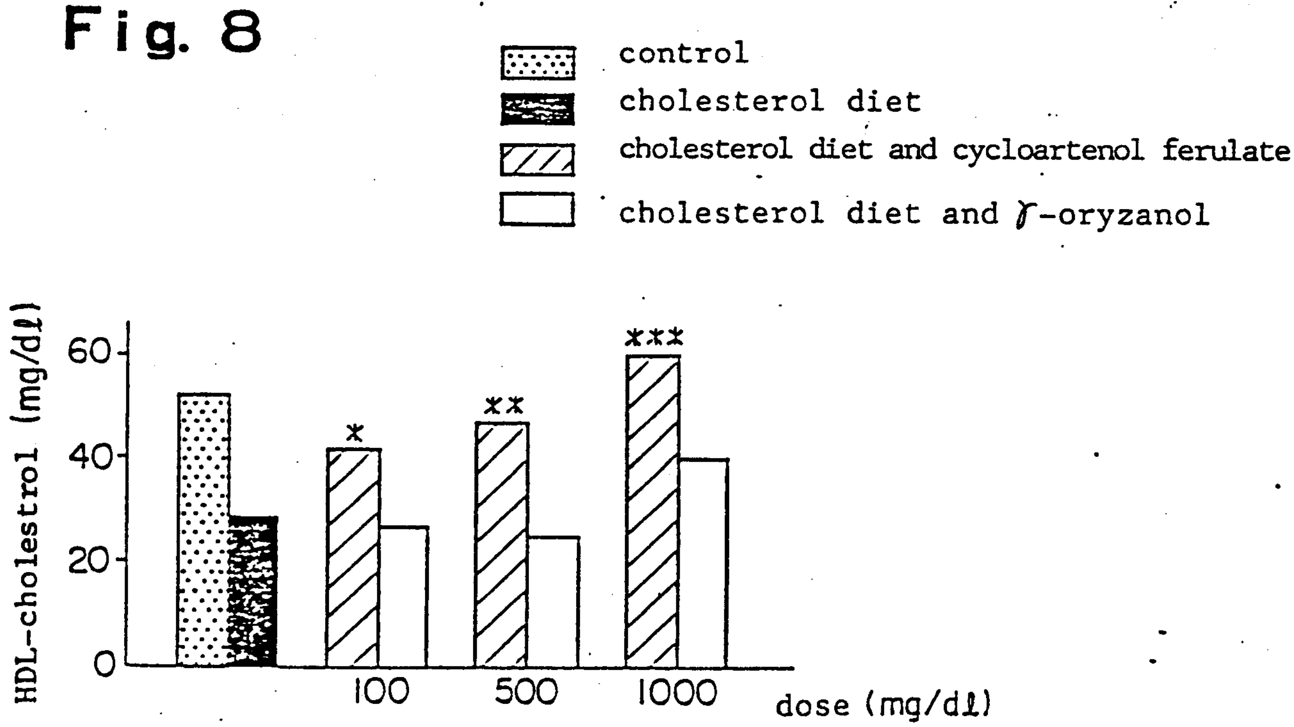


significant difference from cholesterol diet
*$P<0.05$ **$P<0.01$ ***$P<0.001$

## Fig.9

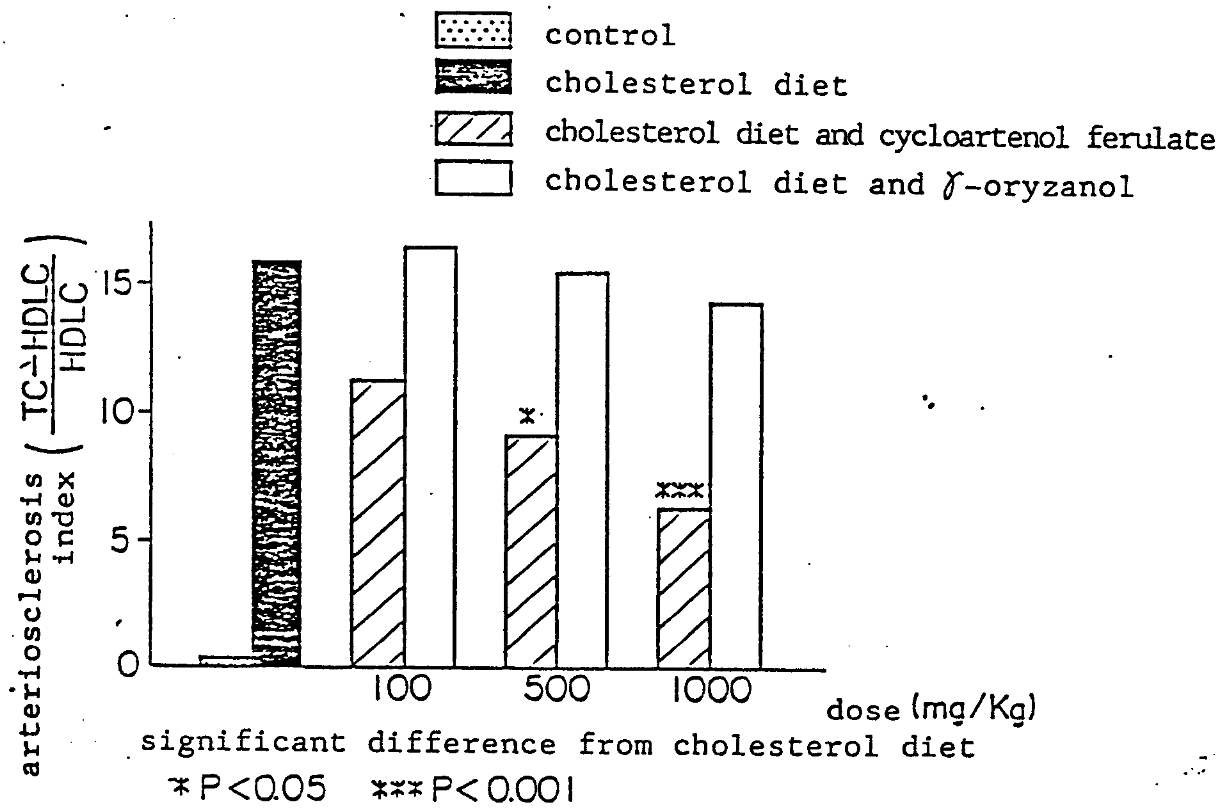


significant difference from cholesterol diet

* $P<0.05$ *** $P<0.001$

## Fig. 12

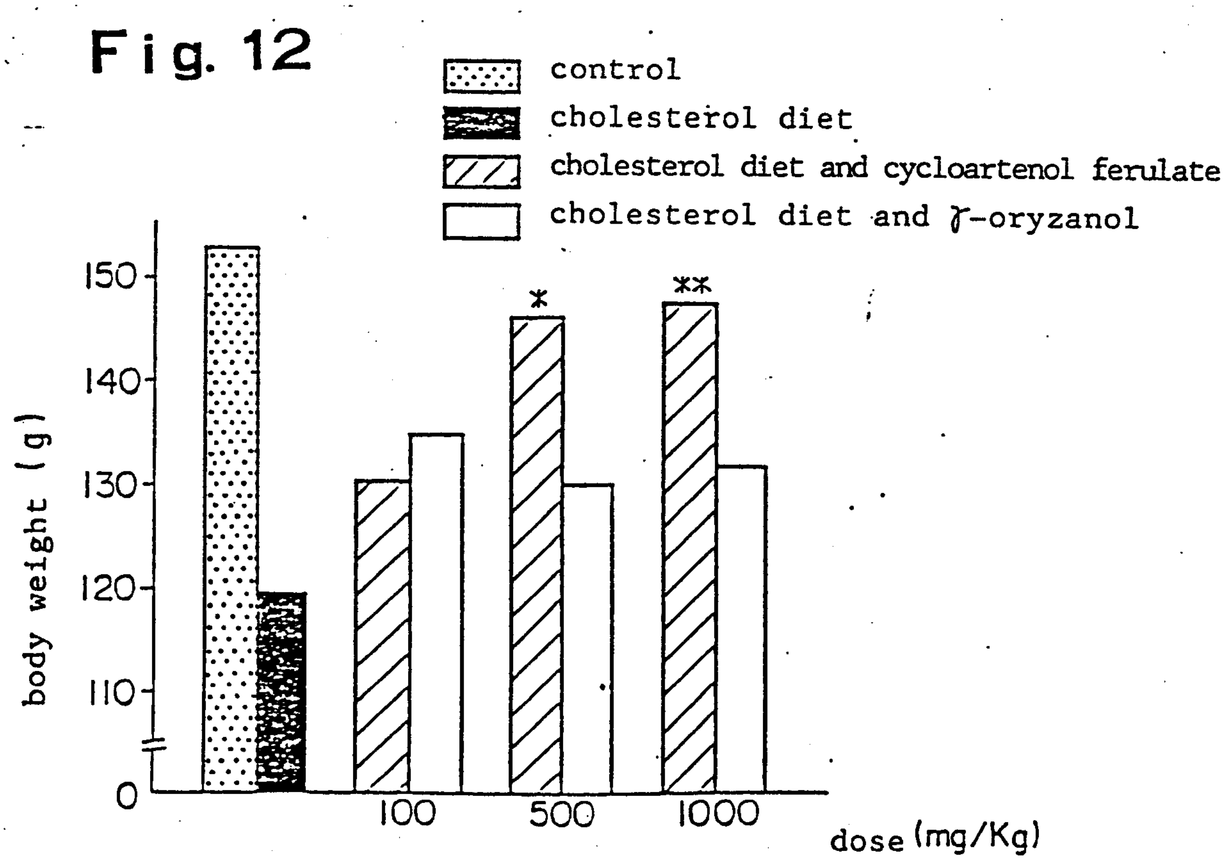


significant difference from cholesterol diet

* $P<0.05$ ** $P<0.01$

Fig. 10

control
cholesterol diet
cholesterol diet and cycloartenol ferulate
cholesterol diet and γ-oryzanol

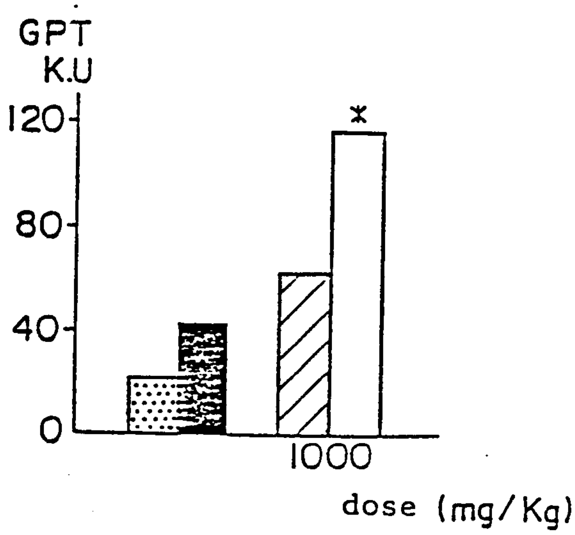


Fig. 11

control
cholesterol diet
cholesterol diet and cycloartenol ferulate
cholesterol diet and γ-oryzanol

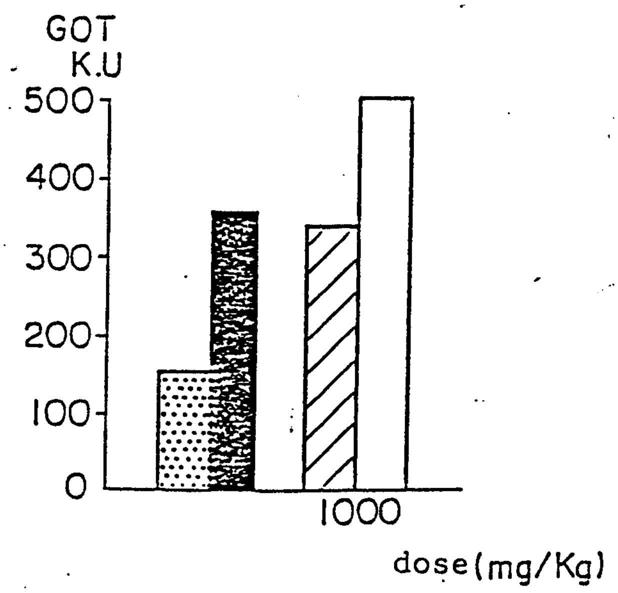


significant difference from cholesterol diet
$*P<0.05$

Fig.13

Triton control

Tritonl
and cycloartenol ferulate
(10mg/Kg i.v.)

Triton and γ-oryzanol
(10mg/Kg i.v.)

* P<0.05
significant difference
from Triton control

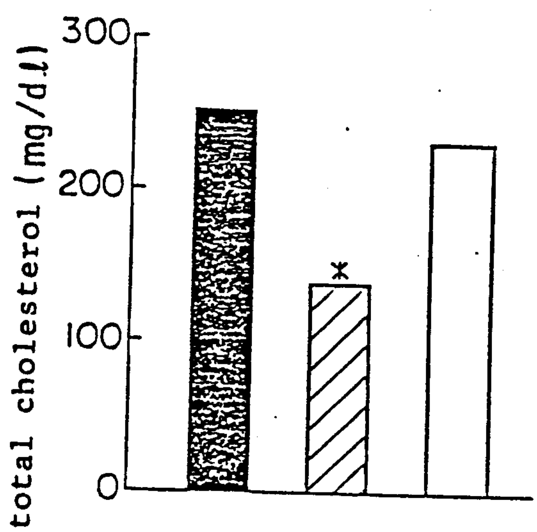


Fig.14

Triton control

Tritonl
and cycloartenol ferulate
(10mg/Kg i.v.)

Triton and γ-oryzanol
(10mg/Kg i.v.)

* P<0.05
significant difference
from Triton control

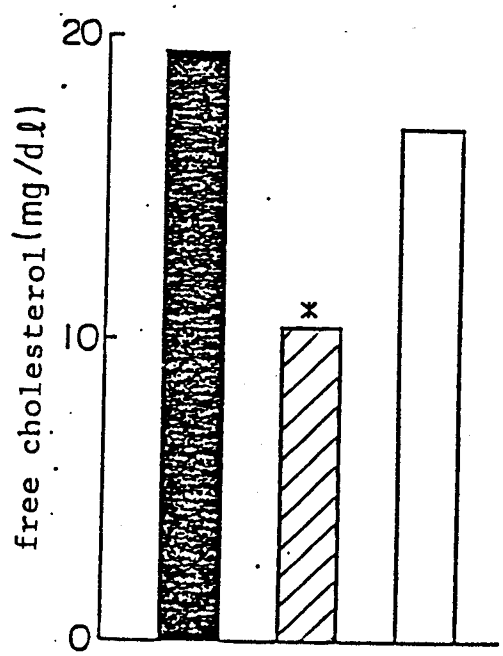

# Fig.15

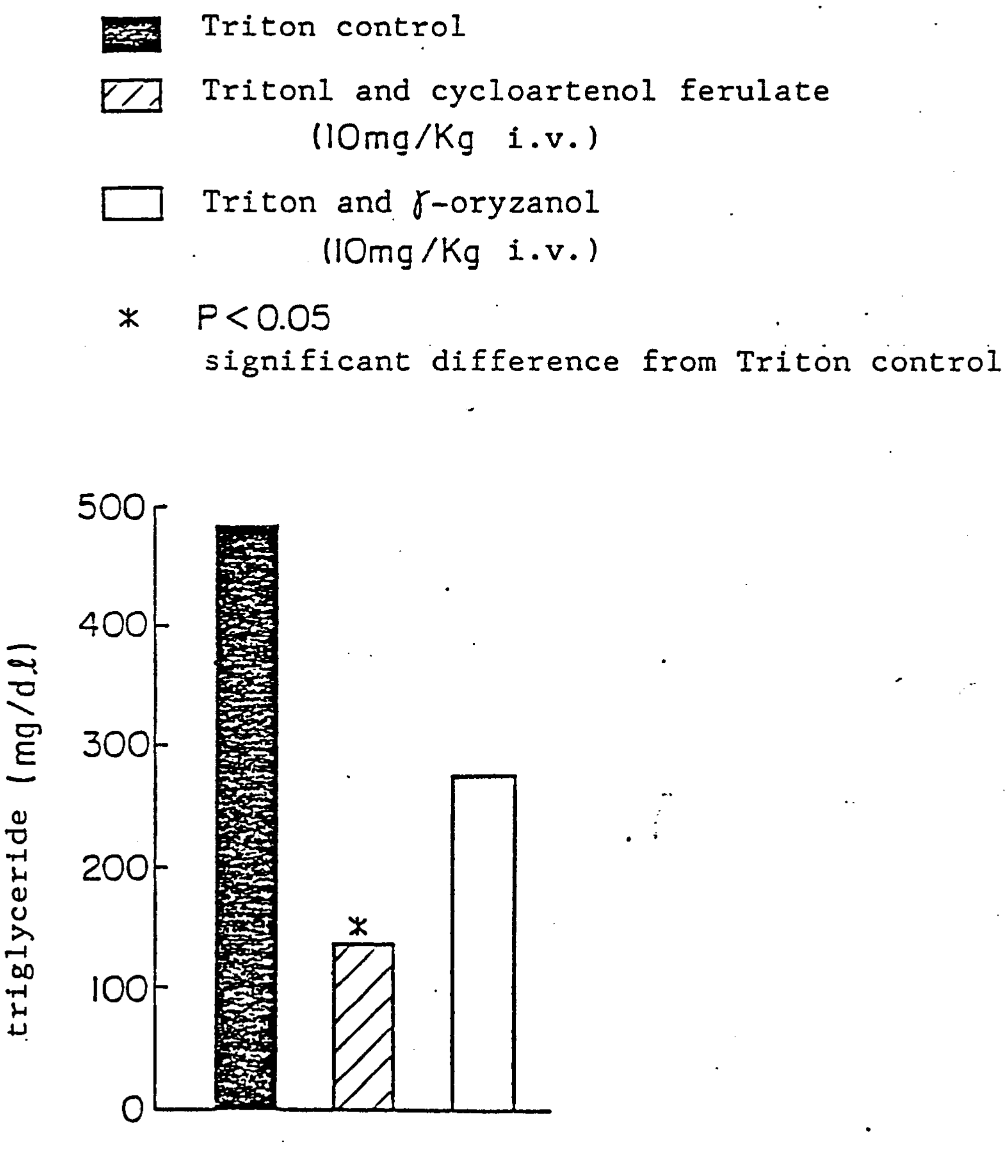
Triton control
Tritonl and cycloartenol ferulate
(10mg/Kg i.v.)
Triton and γ-oryzanol
(10mg/Kg i.v.)
* P<0.05
significant difference from Triton control
500
400
300
200
100
0
triglyceride (mg/dℓ)
*

# INTERNATIONAL SEARCH REPORT

0222912

International Application No. PCT/JP 85/00286

**I. CLASSIFICATION OF SUBJECT MATTER** (if several classification symbols apply, indicate all) [3]

According to International Patent Classification (IPC) or to both National Classification and IPC

Int. $Cl^4$ C07J51/00, A61K31/575

**II. FIELDS SEARCHED**

Minimum Documentation Searched [4]

| Classification System | Classification Symbols |
|---|---|
| I P C | C07J51/00, A61K31/575 |

Documentation Searched other than Minimum Documentation to the Extent that such Documents are Included in the Fields Searched [5]

**III. DOCUMENTS CONSIDERED TO BE RELEVANT** [14]

| Category* | Citation of Document, [16] with indication, where appropriate, of the relevant passages [17] | Relevant to Claim No. [18] |
|---|---|---|
| X | JP, A, 57-149248 (Tsuchiya Tomodairo) 14 September 1982 (14. 09. 82) (Family: None) | 1 |

* Special categories of cited documents: [15]
- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step
- "Y" document of particular relevance; the claimed invention cannot be considered to involve an inventive step when the document is combined with one or more other such documents, such combination being obvious to a person skilled in the art
- "&" document member of the same patent family

**IV. CERTIFICATION**

| Date of the Actual Completion of the International Search [2] | Date of Mailing of this International Search Report [2] |
|---|---|
| August 15, 1985 (15. 08. 85) | August 26, 1985 (26. 08. 85) |
| **International Searching Authority** [1] | **Signature of Authorized Officer** [20] |
| Japanese Patent Office | |